# EUROPÄISCHE PATENTSCHRIFT

(11) **EP 3 444 825 B1**
(45) Veröffentlichungstag und Bekanntmachung des Hinweises auf die Patenterteilung: **03.01.2024**
(21) Anmeldenummer: 17186237.8
(22) Anmeldetag: 15.08.2017
(51) Int. Cl.: G16H 40/20

(54) **OPTIMIEREN EINES BETRIEBS EINES MEDIZINISCHEN SYSTEMS**
OPTIMISING THE OPERATION OF A MEDICAL SYSTEM
OPTIMISATION D'UN FONCTIONNEMENT D'UN SYSTÈME MÉDICAL

(43) Veröffentlichungstag der Anmeldung: 20.02.2019
(73) Patentinhaber: Siemens Healthcare GmbH, 91052 Erlangen (DE)
(72) Erfinder: Pickert, Nils, 91056 Erlangen (DE); Weingarten, Markus, 96049 Bamberg (DE)
(74) Vertreter: Siemens Healthineers Patent Attorneys

(56) Entgegenhaltungen:
- EP-A2- 2 682 885
- KR-A- 20160 116 949
- US-A1- 2006 006 999
- US-A1- 2008 147 529
- US-A1- 2008 164 998
- US-A1- 2015 149 330
- US-A1- 2017 177 807

## Beschreibung

Die vorliegende Erfindung betrifft ein Verfahren zum Betreiben eines medizinischen Systems. Darüber hinaus betrifft die vorliegende Erfindung ein entsprechendes medizinisches System.

Bei einem medizinischen System handelt es sich um eine Mehrzahl an Objekten, die an einer medizinischen Prozedur teilhaben oder zumindest vorgesehen sind, daran teilzuhaben. Beispielsweise handelt es sich bei einer Herzklappenoperation um eine derartige medizinische Prozedur. An dieser Herzklappenoperation nehmen beispielsweise ein Chirurg, ein Anästhesist und mehrere Assistenten als Personen teil. An Gerätschaften sind beispielsweise für diese medizinische Prozedur ein Operationstisch, ein C-Bogen-Röntgengerät, ein Anästhesiewagen, ein Monitoraufbau, ein Bestecktisch, ein Ultraschallgerät und dergleichen notwendig. Die Geräte können gegebenenfalls auch untereinander mechanisch oder datentechnisch verbunden sein. All diese Geräte und Personen werden vorliegend als Objekte bezeichnet, die Teil des medizinischen Systems sind.

Viele medizinische Prozeduren werden immer von gleich qualifizierten Personen durchgeführt. Darüber hinaus werden für medizinische Prozeduren gleichen Typs von einer Mannschaft immer die gleichen Geräte verwendet. Ähnliches gilt auch für die eingesetzten Hilfsmittel wie Applikationen, Workflows, Datenverarbeitungsanlagen, Verbrauchsmaterial und dergleichen für die jeweilige medizinische Prozedur.

Die Gesamtheit der Objekte, die an einer medizinischen Prozedur teilnehmen, ist in der Regel sehr umfangreich. Nicht selten kommt es daher vor, dass das eine oder andere Objekt für die medizinische Prozedur vergessen wird oder aus sonstigen Gründen nicht zur Verfügung steht. Dadurch ergeben sich im einfachsten Fall nur Verzögerungen, oder aber auch Gefährdungen der Personen und insbesondere des Patienten.

Aus der Druckschrift US 2008/0147529 A1 ist ein Verfahren zum Nachverfolgen von medizinischen Objekten bekannt. Dabei wird zunächst anhand einer persönlichen Zuordnung zum jeweiligen Patienten festgestellt, welche medizinische Prozedur durchgeführt werden soll. Sodann wird ermittelt, welche Gegenstände für die durchzuführende Prozedur erforderlich sind. Schließlich wird das Vorhandensein der Gesamtheit dieser erforderlichen Gegenstände mittels RFID Tracking nachverfolgt.

Aus der Druckschrift KR 2016 0116949 A ist ein ähnliches Verfahren bekannt, bei dem jedoch nicht anhand der Identität des Patienten sondern anhand einer Prozedur- und Raumplanung einer Klinik festgestellt wird, welche Prozedur durchgeführt werden soll.

Aus der Druckschrift US 2006/0006999 A1 schließlich ist ebenfalls ein Verfahren zum Nachverfolgen von Personen, Objekten oder Messwerten mittels RFID Tracking anhand einer jeweils vorgesehenen Prozedur bekannt.

Die Aufgabe der vorliegenden Erfindung besteht somit darin, ein Verfahren zum Betreiben eines medizinischen Systems bereitzustellen, mit dem eine medizinische Prozedur sicherer oder zielgerichteter durchgeführt werden kann. Darüber hinaus soll ein entsprechendes medizinisches System bereitgestellt werden.

Erfindungsgemäß wird diese Aufgabe durch ein Verfahren und ein medizinisches System gemäß den unabhängigen Ansprüchen gelöst. Vorteilhafte Weiterbildungen der Erfindung ergeben sich aus den Unteransprüchen.

Es wird demnach ein Verfahren zum Betreiben eines medizinischen Systems bereitgestellt, bei dem ein Erfassen von Parameterwerten, die eine aktuelle Situation, in welcher sich das medizinische System befindet, beschreiben. Das medizinische System setzt sich dabei vorzugsweise aus mehreren Objekten zusammen, die miteinander zusammenwirken, um eine entsprechende medizinische Prozedur ausführen zu können. Diese Objekte können jeweils durch einen oder mehrere Parameterwerte gekennzeichnet sein. Ein solcher Parameterwert ist durch ein spezifisches Kennzeichen gegeben, das an dem jeweiligen Objekt angebracht ist. Wird dann der Parameterwert erfasst, kann daraus geschlossen werden, dass das Objekt für die medizinische Prozedur präsent ist. Insgesamt werden mehrere Parameterwerte erfasst, die beispielsweise jeweils für ein Objekt stehen. All diese Parameterwerte charakterisieren eine aktuelle Situation, in der sich das medizinische System befindet. D.h. die Parameterwerte beschreiben den Zustand des medizinischen Systems gegebenenfalls seine Umgebung.

Anschließend erfolgt ein Ermitteln einer Situationskennung für die aktuelle Situation aus den erfassten Parameterwerten. Entsprechend einer Klassifikation wird dabei aus vorzugsweise der Gesamtheit der erfassten Parameterwerte eine Zuordnung zu einer Situationskennung durchgeführt. Eine derartige Situationskennung wäre beispielsweise der Begriff "Herzklappenoperation" für die entsprechende medizinische Prozedur. Es wird also automatisch aus den einzelnen Parameterwerten darauf geschlossen, in welcher Situation sich das medizinische System befindet. Beispielsweise wird aus der Anzahl und Qualifikation des erfassten Personals und den bereitgestellten Gerätschaften automatisch erkannt, dass eine Herzklappenoperation durchgeführt werden soll. Daher erkennt das medizinische System selbst diese Situation.

In einem weiteren Schritt erfolgt ein Überprüfen der Parameterwerte mittels korrespondierender Werte einer Datenbank, aus der die korrespondierenden Werte anhand der ermittelten Situationskennung gewonnen werden. In einer solchen Datenbank sind beispielsweise in einem oder mehreren Datensätzen Parameterwerte zugeordnet zu jeweiligen Situationskennungen hinterlegt. So können beispielsweise gerade für eine Herzklappenoperation entsprechende Parameterwerte in der Datenbank gespeichert sein. Aus der Datenbank können demnach diejenigen Personen, Geräte, Verbrauchsmaterialien, Umgebungsbedingungen und so weiter entnommen werden, die beispielsweise standardmäßig bei einer entsprechenden medizinischen Prozedur eingesetzt werden. Die Datenbank kann aber auch Parameterwerte für ganz spezifische Situationen aufweisen, wenn z.B. Dr. Lopez die Herzklappenoperation durchführt. Ebenso kann ein Parametersatz für Dr. Maier in der Datenbank hinterlegt sein.

Nun erfolgt in einer ersten Alternative ein Ausgeben eines Hinweises, falls mindestens einer der Parameterwerte für die aktuelle Situation nicht mit einem jeweiligen der korrespondierenden Werte der Datenbank übereinstimmt. Wird also beispielsweise erkannt, dass das aktuell präsente Monitorsystem mit zwei Monitoren nicht dem in der Datenbank abgespeicherten Monitorsystem mit vier Monitoren entspricht, kann eine entsprechende Fehlermeldung beziehungsweise ein entsprechender Hinweis ausgegeben werden. Der erfasste Parameterwert steht im vorliegenden Beispiel für das 2-Monitor-System, während der korrespondierende Wert in der Datenbank für die ermittelte aktuelle Situation beziehungsweise Situationskennung ein 4-Monitor-System fordert. Daher wird ein entsprechender Hinweis akustisch oder optisch von dem medizinischen System ausgegeben, sodass das Monitorsystem gegebenenfalls ausgetauscht werden kann.

In ähnlicher Weise kann auch ein Hinweis von dem medizinischen System ausgegeben werden, falls beispielsweise ein Ultraschallgerät aktuell nicht vorhanden ist, während es standardmäßig bei einer medizinischen Prozedur, der die ermittelte Situationskennung zugeordnet ist, laut Datenbank vorhanden sein müsste. Nach Ausgabe des Hinweises kann das fehlende Ultraschallgerät hinzugenommen werden, um das medizinische System entsprechend zu ergänzen.

Alternativ zu dem Ausgeben eines Hinweises kann bei dem erfindungsgemäßen Verfahren ein automatisches Einstellen eines der Parameterwerte entsprechende dem korrespondierenden Wert aus der Datenbank erfolgen. Es wird also nach dem Erfassen einer aktuellen Situation einer oder mehrere bestimmte Parameterwerte des medizinischen Systems beziehungsweise der Umgebung des medizinischen Systems automatisch entsprechend dem korrespondierenden Wert der Datenbank eingestellt. Wenn also beispielsweise aufgrund der Parameterwerte eine Situation entsprechend der Situationskennung "Gallensteinoperation" erkannt wurde, wird das Ultraschallgerät automatisch auf die in der Datenbank gespeicherten Werte für diese Situation eingestellt. In vorteilhafter Weise bietet das erfindungsgemäße Verfahren somit die Möglichkeit, automatisch Abweichungen von Standardsituationen zu erkennen oder entsprechende Standardwerte einzustellen. Damit sinkt das Gefahrenpotential beziehungsweise erhöht sich der Bedienkomfort bei medizinischen Prozeduren.

Vorzugsweise beinhalten die Parameterwerte Informationen, welche elektronischen Objekte zu dem medizinischen System kombiniert sind. Es werden also in diesem Beispiel zumindest diejenigen elektronischen Geräte und Apparate erkannt, welche zu dem medizinischen System gehören. Derartige elektronische Objekte können beispielsweise über drahtlose Kommunikationsverbindungen registriert werden. Solche elektronischen Objekte sind beispielsweise ein C-Bogen-Röntgengerät, ein elektronisch verstellbarer OP-Tisch, ein Anästhesiesystem und dergleichen.

Speziell kann vorgesehen sein, dass das Ausgeben des Hinweises dann erfolgt, wenn für die ermittelte Situationskennung die korrespondierenden Werte der Datenbank ergeben, dass bei den zu dem medizinischen System kombinierten Objekten mindestens ein Objekt fehlt. Es wird also die Abwesenheit eines Objekts in dem medizinischen System überprüft, wobei die Datenbank als Referenz herangezogen wird. Auf diese Weise kann zuverlässig verhindert werden, dass für eine spezifische medizinische Prozedur ein wichtiges Gerät oder ein wichtiger Apparat fehlt.

In einer weiteren Ausgestaltung ist beabsichtigt, dass eines der Objekte ein Gerät, ein Werkzeug, ein Datennetzwerk oder eine Person ist. Beispielsweise wird in einer aktuellen Situation, die einer Vorbereitungsphase einer Operation entspricht, festgestellt, dass das Ultraschallgerät nicht im Raum ist. Auf einen entsprechenden Hinweis hin kann das Ultraschallgerät nachträglich beschafft werden. Auch wenn das Ultraschallgerät mit anderen Parametern eingestellt ist als dies die Datenbank fordert, kann ein entsprechender Hinweis ausgegeben werden. Alternativ kann das Ultraschallgerät auch automatisch entsprechend der ermittelten Situation eingestellt werden. Ähnliches gilt auch für Werkzeuge, wie z.B. Messer, Sägen, Hammer, aber auch für Verbrauchsmaterial wie etwa Schrauben, Platten und dergleichen. Falls sie in der erkannten Situation fehlen, wird ein entsprechender Hinweis ausgegeben. Auch kann der Zugang zu einem Datennetzwerk (z.B. Krankenhaussystem) überprüft beziehungsweise eingestellt werden. Ähnliches gilt für Personen, die in der aktuellen Situation erfasst beziehungsweise standardmäßig vorhanden sein sollten. Beispielsweise ist es günstig, dass in einer vorgegebenen Situation immer eine dritte Operationsschwester bereitsteht. Das Fehlen kann auch hier entsprechend angezeigt werden.

Weiterhin kann bei dem erfindungsgemäßen Verfahren vorgesehen sein, dass jedes der Objekte ein RFID-Kennzeichenelement besitzt, und das Erfassen der Parameterwerte ein Auslesen sämtlicher RFID-Kennzeichenelemente der Objekte des medizinischen Systems umfasst. RFID-Kennzeichenelemente können drahtlos ausgelesen werden. Außerdem können sie eine gewisse Menge an Informationen enthalten, die für das medizinische System von Bedeutung sind. Insbesondere können sie diejenigen Parameterwerte enthalten beziehungsweise übertragen, die für das Überprüfen in Bezug auf die Situationskennung notwendig sind.

Des Weiteren kann vorgesehen sein, dass die korrespondierenden Werte der Datenbank aus einem Datensatz stammen, der manuell gewählt wird. Wird also beispielsweise eine bestimmte Situation, in der sich das medizinische System befindet, erkannt, so können mehrere Datensätze zur Auswahl gestellt werden. Speziell können mehrere Datensätze zur Verfügung gestellt werden, wenn eine Situation wie etwa die Herzklappenoperation festgestellt wurde. In diesem Fall kann der Chirurg denjenigen Datensatz auswählen, der für ihn subjektiv der passende ist. So kann beispielsweise ein Datensatz verwendet werden, der immer bei einer Herzklappenoperation in einem renommierten Krankenhaus verwendet wird.

Besonders vorteilhaft ist darüber hinaus, wenn bezüglich der Situationskennung von dem medizinischen System Datensätze gelernt werden, und die korrespondierenden Werte in der Datenbank aus den Datensätzen gebildet werden. So kann beispielsweise eine medizinische Prozedur mehrfach wiederholt und die aufgenommenen Parameterwerte entsprechend gelernt werden. Wurde die medizinische Prozedur beispielsweise fünfmal durchgeführt, so kann aus den jeweils aufgenommenen Datensätzen ein gegebenenfalls gemittelter Datensatz gebildet und in der Datenbank abgespeichert werden. Dieser Datensatz steht dann für zukünftige Prozeduren zur Verfügung.

Zudem kann vorgesehen sein, dass eines der Objekte einem Objekttyp zugeordnet ist, wobei dieses Objekt des Objekttyps in einer Betriebsprozedur des medizinischen Systems nur einmalig benutzbar ist, und mittels eines Warenwirtschaftssystems automatisch überprüft wird, ob ein weiteres Objekt dieses Objekttyps für eine weitere Betriebsprozedur vorrätig ist. So können beispielsweise einmalig verwendbare Verbrauchsmaterialien wie Tupfer, aber auch stets neu zu desinfizierende Instrumente, wie etwa Skalpelle, in der jeweiligen Situation beispielsweise hinsichtlich ihres Fehlens überprüft werden. Gleichzeitig kann durch deren Erfassung mittels des Warenwirtschaftssystems auch deren Bestand aktualisiert werden. Falls die jeweiligen Objekte dann nicht mehr oder nur in geringer Anzahl vorrätig sind, kann automatisch eine entsprechende Bestellung generiert werden.

Die oben dargestellte Aufgabe wird erfindungsgemäß auch gelöst durch ein medizinisches System, aufweisend eine Erfassungseinrichtung zum Erfassen von Parameterwerten, die durch spezifische Kennzeichen gegeben sind, die an einem jeweiligen Objekt angebracht sind, die eine aktuelle Situation, in welcher sich das medizinische System befindet, beschreiben, und eine Datenverarbeitungseinrichtung zum Ermitteln einer Situationskennung für die aktuelle Situation aus den erfassten Parameterwerten, wobei die Datenverarbeitungseinrichtung ausgebildet ist zum Überprüfen der Parameterwerte mittels korrespondierender Werte einer Datenbank, aus der die korrespondierenden Werte anhand der ermittelten Situationskennung gewonnen werden, sowie
eine Steuereinrichtung zum Ausgeben eines Hinweises, falls mindestens einer der Parameterwerte nicht mit einem jeweiligen der korrespondierenden Werte übereinstimmt oder zum automatischen Einstellen eines der Parameterwerte entsprechend dem korrespondierenden Wert aus der Datenbank.

Die oben im Zusammenhang mit dem erfindungsgemäßen Verfahren erläuterten Vorteile und Variationsmöglichkeiten lassen sich auch auf das medizinische System übertragen. Dabei stellen die geschilderten Verfahrensmerkmale funktionelle Merkmale entsprechender Mittel wie insbesondere der Erfassungseinrichtung, der Datenverarbeitungseinrichtung und der Ausgabeeinrichtung dar.

In einer speziellen Ausgestaltung kann das medizinische System ein Diagnosegerät und insbesondere ein Röntgengerät aufweisen. Gegebenenfalls können als Parameterwerte nicht nur das Gerät selbst, sondern auch dessen Einstellungen von dem medizinischen System erfasst werden. So kann beispielsweise die Dosis für eine Röntgenaufnahme als Parameterwert erfasst werden. In diesem Fall lässt sich die Dosis anhand der Datenbank überprüfen und gegebenenfalls auch automatisch ändern.

Die vorliegende Erfindung wird nun anhand der beigefügten Zeichnungen näher erläutert, in denen zeigen:
- FIG 1: eine schematische Ansicht eines medizinischen Systems in einer beispielhaften Situation; und
- FIG 2: ein Diagramm zum Ablauf eines erfindungsgemäßen Verfahrens.

Die nachfolgend näher geschilderten Ausführungsbeispiele stellen bevorzugte Ausführungsformen der vorliegenden Erfindung dar. Dabei ist zu beachten, dass die einzelnen Merkmale nicht nur in den geschilderten Merkmalskombinationen, sondern auch in Alleinstellung oder in anderen sinnvollen technischen Kombinationen realisiert werden können.

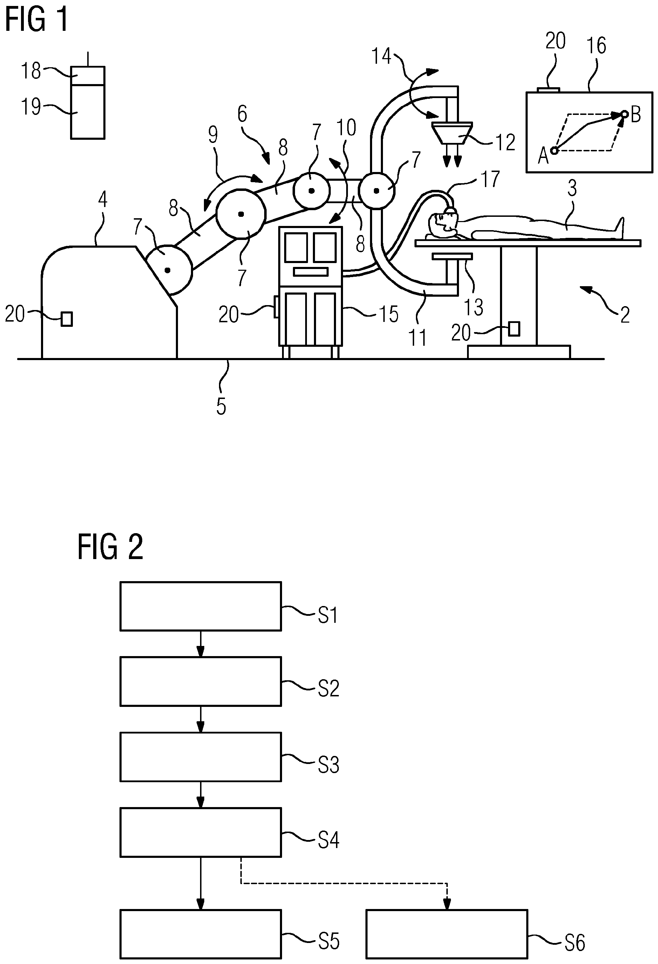

FIG 1 zeigt schematisch ein medizinisches System mit einem C-Bogen-Röntgengerät 1 und einem Patiententisch 2, auf dem sich ein Patient 3 befindet. Das C-Bogen-Röntgengerät 1 besitzt einen Sockel 4, der z.B. drehbar auf dem Boden 5 befestigt ist. Des Weiteren besitzt das C-Bogen-Röntgengerät 1 einen Gelenkarm 6, der mit seinem proximalen Ende an dem Sockel 4 schwenkbar und/oder drehbar gelagert ist. Der Gelenkarm 6 besitzt beispielsweise mehrere Gelenke 7 und zwischen jeweils zweien dieser Gelenke 7 einen jeweiligen Armabschnitt 8. Die Anzahl der Gelenke 7 beziehungsweise Armabschnitte 8 kann beliebig sein. Der Gelenkarm 6 erlaubt Bewegungen 9, 10 in verschiedenen Raumrichtungen. Beispielsweise besitzt das gesamte C-Bogen-Röntgengerät acht Bewegungsachsen, um entsprechend komplexe Bewegungen durchzuführen.

Am distalen Ende des Gelenkarms 6 ist ein C-Bogen 11 angeordnet. An dem einen Ende des C-Bogens 11 befindet sich eine Röntgenstrahlungsquelle 12 und an dem andere Ende ein Detektor 13. Der C-Bogen 11 kann beispielsweise eine Angulation 14 vollführen.

In dem Raum (z.B. Operationssaal) befindet sich außerdem in unmittelbarer Nähe des C-Bogen-Röntgengeräts 1 beziehungsweise des Patiententisches 2 mit dem Patienten 3 ein Anästhesie- oder Beatmungsgerät 15. Ein Schlauch 17 führt von dem Beatmungsgerät 15 zu dem Patienten 3.

Das Beatmungsgerät 15 und der Schlauch 17 stehen stellvertretend für alle möglichen Objekte, die die gesamte aktuelle Situation, in der sich das medizinische System befindet, mitbeschreiben. Als derartige Objekte gelten auch andere Geräte beziehungsweise Versorgungssysteme (einschließlich Kabel und Schläuche) sowie Personen.

Weiterhin ist an dem C-Bogen-Röntgengerät 1 oder in dem Raum (z.B. Operationssaal) beispielsweise eine Bedienschnittstelle 16 (z.B. Monitor) angeordnet. Diese Bedienschnittstelle 16 informiert beispielsweise über die aktuelle medizinische Prozedur, über die vorhandenen Objekte, über fehlende Objekte oder dergleichen.

Das medizinische System umfasst außerdem eine Erfassungseinrichtung 18 mit daran angeschlossener Datenverarbeitungseinrichtung 19. Mit der Erfassungseinrichtung 18 ist das medizinische System in der Lage, die einzelnen Objekte zu erfassen. Hierzu kann die Erfassungseinrichtung 18 beispielsweise eine Kamera aufweisen, und die Objekte werden durch Bildverarbeitung in der Datenverarbeitungseinrichtung 19 ermittelt. Die Erfassungseinrichtung 18 kann aber auch eine beliebige andere Sensorik aufweisen, um die einzelnen Objekte 1, 2, 15, 16, und so weiter zu erfassen. Die Erfassung kann drahtlos oder drahtgebunden erfolgen. Beispielsweise ist die Erfassungseinrichtung 18 mit einem oder mehreren Objekten des medizinischen Systems über ein Datennetz verbunden.

Konkret kann jedes Objekt, das erfasst werden soll, beispielsweise ein RFID-Kennzeichenelement 20 aufweisen. Damit kann die Erfassungseinrichtung 18 gegebenenfalls nicht nur den Typ des jeweiligen Objekts, sondern auch dessen Position als jeweilige Parameterwerte ermitteln. Unter Umständen besitzt die Erfassungseinrichtung 18 aber auch zusätzliche Sensorik oder Datenschnittstellen, mit denen weitere Parameterwerte, wie etwa die Temperatur, die Helligkeit, die Luftfeuchtigkeit und dergleichen erfasst werden können, um die aktuelle Situation, in welcher sich das medizinische System befindet, ausführlicher beschreiben zu können.

Die von der Erfassungseinrichtung 18 erfassten Parameterwerte, die beispielsweise den Typ des erfassten Objekts und dessen Position betreffen, werden in der Datenverarbeitungseinrichtung 19 weiterverarbeitet. Beispielsweise ist hierbei eine Klassifikation vorgesehen, mit der aus den erfassten Parameterwerten eine Situation beziehungsweise Situationskennung ermittelt wird. Die Situationskennung, wie etwa der Begriff "Herzklappenoperation" charakterisiert die Situation.

Falls das System nun die Situation ausreichend erkannt hat, kann es aus einer beispielsweise in die Datenverarbeitungseinrichtung 19 integrierten Datenbank eine oder mehrere entsprechende Datensätze aufrufen beziehungsweise bereitstellen. Die Datenbank kann aber auch beispielsweise über ein Datennetz von extern zur Verfügung gestellt werden. Auf der Basis der Situationskennung können dann beispielsweise bestimmte Parameter eines C-Bogen-Röntgengeräts 1 fest eingestellt werden. So kann beispielsweise bei einer bestimmten medizinischen Prozedur, die das System erkannt hat, immer ein "Organprogramm" des C-Bogen-Röntgengeräts 1 eingestellt werden. In diesem Fall wäre beispielsweise die Dosis und/oder der Kontrast vorgegeben, um eine optimale Bildqualität beziehungsweise ein gewünschtes Aussehen zu erreichen.

In einer anderen Ausführungsform schlägt das medizinische System beispielsweise eine oder mehrere Datensätze von vorbestimmten Ärzten vor. Auf diese Weise kann der behandelnde Arzt dann selbst entscheiden, ob und gegebenenfalls welches Programm seines Kollegen er verwenden will. Gegebenenfalls werden auch im Sinne eines Assistenzsystems einzelne Schritte von dem medizinischen System vorgeschlagen, wenn der entsprechende Datensatz gewählt wurde.

Unter Umständen zeigt das medizinische System auch Datensätze zur Auswahl an, die verschiedene Richtwerte besitzen. So können beispielsweise die Gesamtröntgendosen für die einzelnen Datensätze angegeben werden. Auf diese Weise ist es möglich, beispielsweise einen Datensatz zu wählen, bei dem das medizinische System mit einer geringeren Röntgendosis auskommt als mit einem anderen Datensatz. Anhand solcher Richtwerte kann der behandelnde Arzt subjektiv entscheiden, welche Parametereinstellungen beziehungsweise Parameterwerte in einer bestimmten Situation optimal sind. Diese Parameterwerte müssen nicht zwangsläufig diejenigen sein, die zu einem besten von allen möglichen Ergebnissen führen. So kann es beispielsweise Ziel eines behandelnden Arztes sein, einen anderen Parameter als die Röntgendosis zu optimieren.

In einer weiteren Ausführungsform kann es sich bei dem medizinischen System um ein lernendes System handeln. So kann das medizinische System bei mehreren medizinischen Prozeduren gleichen Typs lernen, welche Parameterwerte in dieser Situation verwendet beziehungsweise eingestellt werden. So wird beispielsweise beim Austauschen einer Herzklappe immer ein Ultraschallgerät verwendet. Falls dieser Umstand vom System gelernt und entsprechend in der Datenbank abgespeichert ist, kann bei dem Überprüfen der aktuellen Parameterwerte festgestellt werden, dass ein solches Ultraschallgerät in der aktuellen Situation fehlt. Entsprechend kann beispielsweise auf dem Monitor 16 (vergleiche FIG 1) ein geeigneter Hinweis gegeben werden.

Bei einer alternativen Anwendung werden Einstellungen eines oder mehrerer Objekte beziehungsweise Geräte vorab durchgeführt. So können beispielsweise einzelne Geräte so konfiguriert werden, wie sie bei den letzten fünf Prozeduren konfiguriert waren. Beispielsweise erhöht ein bestimmter Arzt den Kontrast bei einer vorgegebenen Operation immer um zwei Stufen. Sobald das System dies gelernt hat, stellt es den Kontrast automatisch um zwei Stufen höher, wenn die entsprechende Situation (insbesondere der Arzt) erkannt wurde. Ähnliches kann für übergewichtige Patienten gelten, wenn das System gelernt hat, dass dort stets die Dosis beispielsweise um 10 Prozent erhöht wurde.

Besonders leicht kann das System die Objekte (z.B. Personen, Werkzeuge, etc.) erkennen, wenn diese mit einem RFID-Kennzeichenelement beziehungsweise RFID-Tag versehen sind. Auch andere Sensorik kann eingesetzt werden, um die aktuelle Situation zu erkennen. Insbesondere können auch Netzwerkinformationen dafür herangezogen werden.

Hat das lernende System beispielsweise erkannt, dass bei einer bestimmten Prozedur drei Nadeln und 15 Tupfer verwendet werden, und am nächsten Tag wieder eine derartige Prozedur ansteht, so kann diese Information für ein Warenwirtschaftssystem genutzt werden. Gegebenenfalls werden diese Verbrauchsmaterialien, falls diese nicht mehr ausreichend auf Lager sind, automatisch nachbestellt.

FIG 2 zeigt ein erfindungsgemäßes Verfahren zum Betreiben eines medizinischen Systems als Blockdiagramm in mehreren Einzelschritten. In einem ersten Schritt S1 erfolgt ein Erfassen von Parameterwerten, die eine aktuelle Situation, in welcher sich das medizinische System befindet, beschreiben. Dabei werden beispielsweise Kennzeichen von Objekten, Positionen von Objekten, Einstellungen von Geräten und andere Sensorwerte als Parameterwerte aufgenommen. In einem zweiten Schritt S2 erfolgt ein Ermitteln einer Situationskennung für die aktuelle Situation aus den erfassten Parameterwerten. Es wird also beispielsweise automatisch aufgrund der erfassten Objekte die Situation "Herzklappenoperation" festgestellt.

In einem optionalen Schritt S3 erfolgt ein Auswählen eines Datensatzes, der der ermittelten Situationskennung zugeordnet ist. Gegebenenfalls stellt nämlich eine Datenbank mehrere derartige Datensätze zur Verfügung, die manuell beispielsweise durch den behandelnden Arzt ausgewählt werden können.

Anschließend erfolgt in einem vierten Schritt S4 ein Überprüfen der Parameterwerte mittels korrespondierender Werte einer Datenbank, aus der die korrespondierenden Werte anhand der ermittelten Situationskennung gewonnen werden. Der ermittelten Situationskennung sind also beispielsweise einer oder mehrere Datensätze mit Parameterwerten zugeordnet, die mit den erfassten Parameterwerten verglichen werden.

Schließlich kann in einem Schritt S5 ein Ausgeben eines Hinweises erfolgen, falls mindestens einer der Parameterwerte für die aktuelle Situation nicht mit einem jeweiligen der korrespondierenden Werte der Datenbank übereinstimmt. Falls also beispielsweise in der aktuellen Situation das Ultraschallgerät fehlt und damit der entsprechende Parameterwert von dem Referenzwert (korrespondierenden Wert) der Datenbank abweicht, so erfolgt beispielsweise auf dem Monitor 16 (vergleiche FIG 1) eine entsprechende Anzeige.

Alternativ oder zusätzlich zu dem Schritt S5 erfolgt in einem sechsten Schritt S6 ein automatisches Einstellen eines der Parameterwerte entsprechend dem korrespondierenden Wert aus der Datenbank. Es wird also nicht (nur) eine Anzeige zur Verfügung gestellt, sondern es wird aktiv eine Einstellung an einem Objekt beziehungsweise Gerät vorgenommen. So wird beispielsweise der Kontrast eines C-Bogen-Röntgengeräts um 20 Prozent automatisch erhöht.

## Patentansprüche

1. Verfahren zum Betreiben eines medizinischen Systems **gekennzeichnet durch**
- Erfassen (S1) von Kennzeichen von Objekten (1, 2, 15, 16), welche zu dem medizinischen System kombiniert sind,
- Ermitteln (S2) einer einer medizinischen Prozedur entsprechenden Situationskennung aus den erfassten Kennzeichen der Objekte,
- Überprüfen (S4) der Kennzeichen der Objekte mittels korrespondierender Werte einer Datenbank, aus der die korrespondierenden Werte anhand der ermittelten Situationskennung gewonnen werden, und
- Ausgeben (S5) eines Hinweises, wenn für die ermittelte Situationskennung die korrespondierenden Werte der Datenbank ergeben, dass bei den zu dem medizinischen System kombinierten Objekten (1, 2, 15, 16) mindestens ein Objekt fehlt, oder
- automatisches Einstellen (S6) eines Geräts, das einem der Objekte des medizinischen Systems entspricht, entsprechend einem der korrespondierenden Werte aus der Datenbank.

2. Verfahren nach Anspruch 1, wobei eines der Objekte (12, 15, 16) ein Gerät, ein Datennetzwerk, ein Werkzeug oder eine Person ist.

3. Verfahren nach einem der Ansprüche 1 bis 2, wobei jedes der Objekte (1, 2, 15, 16) ein RFID-Kennzeichenelement (20) besitzt, und das Erfassen (S1) der Kennzeichen ein Auslesen sämtlicher RFID-Kennzeichenelemente (20) der Objekte (1, 2, 15, 16) des medizinischen Systems umfasst.

4. Verfahren nach einem der vorhergehenden Ansprüche, wobei die korrespondierenden Werte der Datenbank aus einem Datensatz stammen, der manuell gewählt (S3) wird.

5. Verfahren nach einem der vorhergehenden Ansprüche, wobei bezüglich der Situationskennung von dem medizinischen System ein Datensatz gelernt wird, und die korrespondierenden Werte in der Datenbank aus dem Datensatz gebildet werden.

6. Verfahren nach einem der vorhergehenden Ansprüche, wobei eines der Objekte (1, 2, 15, 16) einem Objekttyp zugeordnet ist, der in einer Betriebsprozedur des medizinischen Systems nur einmalig benutzbar ist und mittels eines Warenwirtschaftssystems automatisch überprüft wird, ob ein weiteres Objekt dieses Objekttyps für eine weitere Betriebsprozedur vorrätig ist.

7. Medizinisches System
**gekennzeichnet durch**
- eine Erfassungseinrichtung (18) zum Erfassen (S1) von Kennzeichen von Objekten (1, 2, 15, 16), welche zu dem medizinischen System kombiniert sind, und
- eine Datenverarbeitungseinrichtung (19) zum Ermitteln (S2) einer einer medizinischen Prozedur entsprechenden Situationskennung aus den erfassten Kennzeichen der Objekte, wobei
- die Datenverarbeitungseinrichtung (19) ausgebildet ist zum Überprüfen (S4) der Kennzeichen der Objekte mittels korrespondierender Werte einer Datenbank, aus der die korrespondierenden Werte anhand der ermittelten Situationskennung gewonnen werden, sowie
- eine Steuereinrichtung zum Ausgeben eines Hinweises, wenn für die ermittelte Situationskennung die korrespondierenden Werte der Datenbank ergeben, dass bei den zu dem medizinischen System kombinierten Objekten (1, 2, 15, 16) mindestens ein Objekt fehlt oder zum automatischen Einstellen eines Geräts, das einem der Objekte des medizinischen Systems entspricht, entsprechend dem korrespondierenden Wert aus der Datenbank.

8. Medizinisches System nach Anspruch 7, das ein Diagnosegerät und insbesondere ein Röntgengerät (1) aufweist.

## Claims

1. Method for operating a medical system
**characterised by**
- acquiring (S1) ID tags of objects (1, 2, 15, 16) which are combined to form the medical system,
- determining (S2) a situation identifier corresponding to a medical procedure from the acquired ID tags of the objects,
- checking (S4) the ID tags of the objects by means of corresponding values in a database from which the corresponding values are obtained on the basis of the determined situation identifier, and
- outputting (S5) a notification if, for the determined situation identifier, the corresponding values in the database reveal that at least one object is missing from the objects (1, 2, 15, 16) combined to form the medical system, or
- automatically setting (S6) a device, which corresponds to one of the objects of the medical system, according to one of the corresponding values from the database.

2. Method according to claim 1, wherein one of the objects (12, 15, 16) is a device, a data network, a tool or a person.

3. Method according to one of claims 1 to 2, wherein each of the objects (1, 2, 15, 16) has an RFID tag (20) and acquisition (S1) of the ID tags involves reading all the RFID tags (20) of the objects (1, 2, 15, 16) of the medical system.

4. Method according to one of the preceding claims, wherein the corresponding values in the database originate from a data record which is manually selected (S3).

5. Method according to one of the preceding claims, wherein, with regard to the situation identifier of the medical system, a data record is learned and the corresponding values in the database are formed from the data record.

6. Method according to one of the preceding claims, wherein one of the objects (1, 2, 15, 16) is assigned to an object type which can be used only once in an operating procedure of the medical system and an inventory control system automatically checks whether a further object of this object type is in stock for a further operating procedure.

7. Medical system
**characterised by**
- an acquisition device (18) for acquiring (S1) ID tags of objects (1, 2, 15, 16) which are combined to form the medical system, and
- a data processing device (19) for determining (S2) a situation identifier corresponding to a medical procedure from the acquired ID tags of the objects, wherein
- the data processing device (19) is configured to check (S4) the ID tags of the objects by means of corresponding values in a database from which the corresponding values are obtained on the basis of the determined situation identifier, and
- a control device for outputting a notification if, for the determined situation identifier, the corresponding values in the database reveal that at least one object is missing from the objects (1, 2, 15, 16) combined to form the medical system or for automatically setting a device, which corresponds to one of the objects of the medical system, according to the corresponding value from the database.

8. Medical system according to claim 7, which includes a diagnostic device and in particular an X-ray machine (1).

## Revendications

1. Procédé pour faire fonctionner un système médical, **caractérisé par**
- la détection (S1) de caractéristiques d'objets (1, 2, 15, 16), qui sont combinés en le système médical,
- la détermination (S2) à partir des caractéristiques des objets, qui ont été détectées, d'une caractérisation de situation correspondant à une procédure médicale,
- le contrôle (S4) des caractéristiques des objets, au moyen de valeurs correspondantes d'une base de données, à partir de laquelle les valeurs correspondantes ont été obtenues à l'aide de la caractérisation de situation, qui a été déterminée, et
- l'émission (S5) d'une indication si, pour la caractérisation de situation qui a été déterminée, les valeurs correspondantes de la base de données donnent qu'il manque au moins un objet parmi les objets (1, 2, 15, 16) combinés en le système médical, ou
- le réglage (S6) automatique d'un appareil, qui correspond à l'un des objets du système médical, conformément à l'une des valeurs correspondantes de la base de données.

2. Procédé suivant la revendication 1, dans lequel l'un des objets (12, 15, 16) est un appareil, un réseau de données, un outil ou une personne.

3. Procédé suivant l'une des revendications 1 à 2, dans lequel chacun des objets (1, 2, 15, 16) possède un élément (20) caractéristique RFID, et la détection (S1) des caractéristiques comprend une lecture de l'ensemble des éléments (20) caractéristiques RFID des objets (1, 2, 15, 16) du système médical.

4. Procédé suivant l'une des revendications précédentes, dans lequel les valeurs correspondantes de la base de données proviennent d'un ensemble de données que l'on choisit (S3) manuellement.

5. Procédé suivant l'une des revendications précédentes, dans lequel en ce qui concerne la caractérisation de situation, on apprend, par le système médical, un ensemble de données et on forme, à partir de l'ensemble de données, les valeurs correspondantes dans la base de données.

6. Procédé suivant l'une des revendications précédentes, dans lequel l'un des objets (1, 2, 15, 16) est associé à un type d'objet, qui peut être utilisé seulement une fois dans une procédure de fonctionnement du système médical, et on contrôle automatiquement, au moyen d'un système d'économie des marchandises, si un autre objet de ce type d'objet est en réserve pour une autre procédure de fonctionnement.

7. Système médical,
**caractérisé par**
- un dispositif (18) de détection pour la détection (S1) de caractéristiques d'objets (1, 2, 15, 16), qui sont combinés en le système médical, et
- un dispositif (19) de traitement de données pour la détermination (S2), à partir des caractéristiques des objets, qui ont été détectées, d'une caractérisation de situation correspondant à une procédure médicale, dans lequel
- le dispositif (19) de traitement de données est constitué pour le contrôle (S4) des caractéristiques des objets, au moyen de valeurs correspondantes d'une base de données, à partir de laquelle les valeurs correspondantes ont été obtenues à l'aide de la caractérisation de situation, qui a été déterminée, ainsi que
- un dispositif de commande pour l'émission d'une indication si, pour la caractérisation de situation, qui a été déterminée, les valeurs correspondantes de la base de données donnent qu'au moins un objet manque parmi les objets (1, 2, 15, 16) combinés en le système médical ou pour le réglage automatique d'un appareil, qui correspond à l'un des objets du système médical, conformément à la valeur correspondante de la base de données.

8. Système médical suivant la revendication 7, qui a un appareil de diagnostic et, en particulier, un appareil (1) de radiographie aux rayons X.
